# EUROPÄISCHE PATENTSCHRIFT

(11) **EP 0 626 365 B1**
(45) Veröffentlichungstag und Bekanntmachung des Hinweises auf die Patenterteilung: **18.12.1996**
(21) Anmeldenummer: 94104558.5
(22) Anmeldetag: 23.03.1994
(51) Int. Cl.: C07C 55/02, C07C 55/18, C07C 55/20, C07C 55/21

(54) **Schmelzflüssige, aliphatische Dicarbonsäuren**
Molten fluid aliphatic dicarboxylic acids
Acides aliphatiques dicarboxyliques fondus liquides

(30) Priorität: 22.05.1993 DE 4317189
(43) Veröffentlichungstag der Anmeldung: 30.11.1994
(73) Patentinhaber: HÜLS AKTIENGESELLSCHAFT, 45764 Marl (DE)
(72) Erfinder: Mumcu, Salih, Dr., D-45772 Marl (DE); Baumann, Franz-Erich, Dr., D-48249 Dülmen (DE)

(56) Entgegenhaltungen:
- US-A- 3 969 253

## Beschreibung

Gegenstand der Erfindung sind schmelzflüssige, aliphatische Dicarbonsäuren mit 8 bis 18 C-Atomen im Kohlenstoffgerüst.

Ein breites Anwendungsgebiet für aliphatische Dicarbonsäuren ist z. B. ihr Einsatz als Rohstoff zur Herstellung von Polyamiden. Ein wohlfeiles Herstellungsverfahren geht von aufgeschmolzenem Diamin und Dicarbonsäure aus. Hierbei werden die Komponenten aufgeschmolzen, vermischt und in der Schmelze polykondensiert. In der Praxis liegt der Nachteil für das Verfahren darin, daß die Dicarbonsäure erst unmittelbar vor der Verwendung aufgeschmolzen werden kann, da sie sich in geschmolzenem Zustand bei längerer Lagerung stark verfärbt. Diese Verfärbung wird dann auf das auskondensierte Polyamid übertragen. Ein derartiges Produkt wird auf dem Markt nicht akzeptiert.

In der Praxis weicht man daher auf eine Polykondensation in wäßriger Phase aus. Dieses Verfahren verlangt, daß im technischen Maßstab große Mengen an Wasser erhitzt und abgedampft werden müssen.

Es ist folglich wünschenswert, Wege zu finden, die Polykondensation in der Schmelze durchzuführen. Aufgabe der Erfindung war es, eine schmelzflüssige, aliphatische Dicarbonsäure zur Verfügung zu stellen, die auch bei längerer Lagerung keine Verfärbung zeigt.

Diese Aufgabe wird durch eine schmelzflüssige, aliphatische Dicarbonsäure erfüllt, die - bezogen auf die Menge an Dicarbonsäure - 0.1 bis 3.0 Gew.-% eines primären Amins enthält. In einer bevorzugten Ausführung weist die Dicarbonsäure einen Gehalt von 0.5 bis 2.0 Gew.-% an primärem Amin auf. Ferner ist es bevorzugt, Dicarbonsäure mit einem Wassergehalt von 0 bis ≦ 5 Gew.-% einzusetzen.

Als primäre Amine werden solche verwendet, die ein aliphatisches, cycloaliphatisches, heterocyclisches oder araliphatisches Kohlenstoffgerüst mit 4 bis 20, vorzugsweise 6 bis 13, C-Atomen besitzen. Bevorzugt werden aliphatische, cycloaliphatische oder araliphatische primäre Amine.

Als Beispiele für primäre Monoamine seien u. a. n-Butylamin, n-Hexylamin, n-Dodecylamin, n-Octadecylamin, Cyclohexylamin, Benzylamin, für di-primäre Amine seien u. a. α,ω-Hexamethylendiamin, α,ω-Octamethylendiamin, α,ω-Decamethylendiamin, α,ω-Dodecamethylendiamin, 1.4-Bis(aminomethyl)cyclohexan, 4.4'-Bis(aminocyclohexyl)methan, Isophorondiamin, 2.2.4-Trimethylhexanmethylendiamin, 2.4.4-Trimethylhexamethylendiamin, 2-Methyl-pentamethylendiamin, 1.4-Bis(aminomethyl)benzol, 1.3-Bis(aminomethyl)benzol oder primär/sekundär gemischte Diamine wie z. B. Triacetondiamin genannt. Die primären Amine können auch als Gemisch enthalten sein.

Bevorzugt enthalten sind di-primäre Amine.

Als aliphatische Dicarbonsäuren kommen solche mit 8 bis 18, vorzugsweise 8 bis 13, C-Atomen im Kohlenstoffgerüst in Frage. Im besonderen seien u. a., Korksäure, Azelainsäure, Sebacinsäure, Undecandisäure, Dodecandisäure, Brassylsäure aufgeführt. Es können auch Gemische von Dicarbonsäuren eingesetzt werden.

Die Herstellung der erfindungsgemäßen schmelzflüssigen, aliphatischen Dicarbonsäure ist nicht Gegenstand der Erfindung. Die Herstellung erfolgt z. B. durch Aufschmelzen der Dicarbonsäure in einem Rührkessel. Die notwendige bzw. erwünschte Menge an primärem Amin wird in die schmelzflüssige Dicarbonsäure gegeben und unter Rühren in der Schmelze gleichmäßig verteilt. Die Herstellung beschränkt sich nicht auf die angeführte Arbeitsmethode.

Jede andere, technisch sinnvolle Arbeitsweise kann mit gleichem Erfolg angewendet werden.

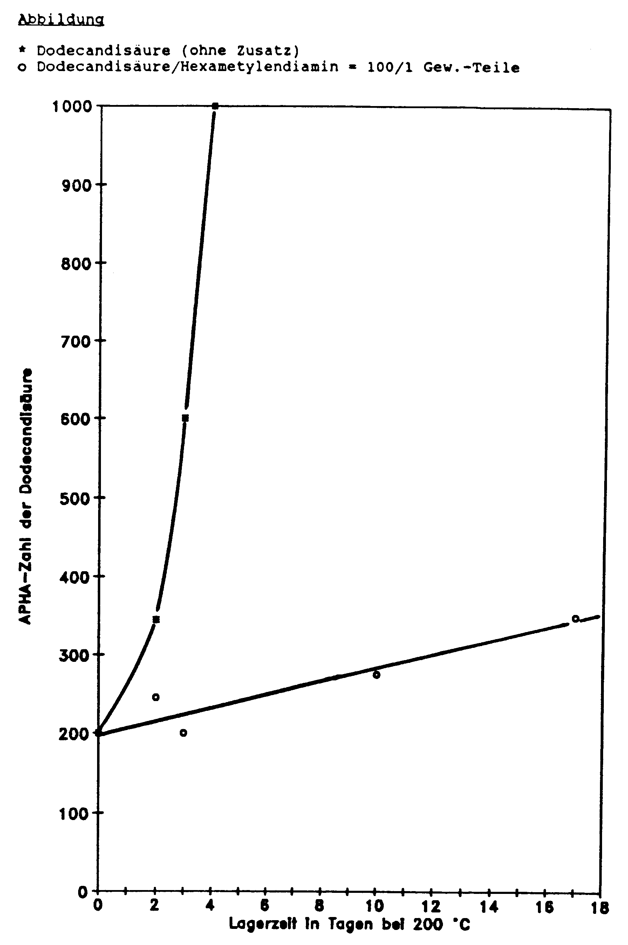

Die erfindungsgemäße schmelzflüssige, aliphatische Dicarbonsäure zeigt eine ausgezeichnete Stabilität der Farbe. Selbst nach mehreren Tagen Lagerung weist die Dicarbonsäure praktisch keine Verfärbung auf. Demgegenüber ist eine geschmolzene Dicarbonsäure, die kein Amin enthält, bereits nach wenigen Tagen stark gelb bis braun verfärbt. Es ist in jedem Fall unerläßlich, daß die schmelzflüssige Dicarbonsäure unter einem inerten Gas, z. B. Stickstoff, gelagert wird. Zur Farbveränderung in Abhängigkeit von der Zeit wird auf die beigefügte Abbildung verwiesen.

Ein wesentliches Anwendungsgebiet für die erfindungsgemäße Dicarbonsäure ist der Einsatz als Rohstoff für die Polyamidherstellung. Aus diesem Grunde können für die Polyamidherstellung übliche Hilfs- und Zusatzstoffe, wie z. B. optische Aufheller, Polykondensationskatalysatoren, hier insbesondere von Phosphor abgeleitete Säuren, enthalten sein. Jedoch ist die Erfindung nicht auf dieses Arbeitsgebiet beschränkt.

Die Bestimmung der Farbzahl gemäß APHA erfolgt an Schmelzen in Küvetten (Höhe: 300 mm - Durchmesser: 25 mm) im Tageslicht, wobei diese Schmelzen gegen wässrige Lösungen von CoCl₂ und K₂PtCl₆ in verschiedenen Konzentrationen als Farbstandards verglichen werden (DIN 53 409).

Die Bestimmung der relativen Lösungsviskosität (ηᵣₑₗ) der Polyamide erfolgt unter Verwendung einer 0,5 gew.-%igen m-Kresol-Lösung bei 25 °C (DIN 53 727/ISO 307).

Zur Bestimmung der Carboxylendgruppen wird 1 g Polyamid in 50 ml Benzylalkolhol unter Stickstoffabdeckung bei 165 °C gelöst. Die Lösezeit beträgt maximal 20 min. Die Lösung wird mit einer Lösung von KOH in Ethylenglykol (0,05 mol KOH/1) gegen Phenolphthalein bis zum Farbumschlag titriert.

Zur Bestimmung der Aminoendgruppen wird 1 g Polyamid in 50 ml m-Kresol bei 25 °C gelöst. Die Lösung wird mit Perchlorsäure potentiometrisch titriert.

Die Bestimmung der Schmelzpunkte wird mittels DSC durchgeführt (ASTM D 34/8).

Die mit Buchstaben gekennzeichneten Beispiele sind nicht erfindungsgemäß.-

### Beispiele

### Beispiel 1

2 kg wasserfreie Dodecandisäure und 20 g n-Hexamethylendiamin werden unter Deoxo-Stickstoff in einem 5 l-Rührautoklaven aufgeschmolzen und 3 Wochen bei 200 °C gelagert. In regelmäßigen Abständen werden über das Bodenventil Proben entnommen und davon die APHA-Zahl bestimmt. Die erhaltenen Ergebnisse sind in der Abbildung dargestellt.

### Beispiel A

Es wird Beispiel 1 wiederholt - jedoch mit dem Unterschied, daß kein Diamin zugesetzt wird. Die erhaltenen Ergebnisse sind in der Abbildung dargestellt.

### Beispiel 2

In einem Kessel (Inhalt: 1 m³) mit Ankerrührer werden 664.6 kg Dodecandisäure (DDS) zusammen mit 6.71 kg Hexamethylendiamin (HMD) vermischt, aufgeschmolzen und bei 165 °C bevorratet. Innerhalb von 14 Stunden werden in einem zweiten Kessel unter Rühren 16.269 kg 85 Gew.-%iges wasserfreies HMD (Δ 140.0 Mol) aufgeschmolzen und in einen Polykondensationskessel gedrückt. Aus dem 1 m³-Kessel werden über eine Dosierpumpe 33.567 kg schmelzflüssiges Produkt, bestehend aus 33.231 kg DDS (Δ 144.3 Mol) und 0.335 kg HMD (Δ 2.886 Mol), dem Polykondensationskessel zugeführt. Es stellt sich im Polykondensationskessel eine Mischtemperatur von 193 °C ein. Unter Eigendruck wird die Schmelze auf 245 °C (22 bar) aufgeheizt und eine Stunde so belassen. Danach wird unter kontinuierlichem Entspannen auf 265 °C (Normaldruck) aufgeheizt. Anschließend wird während 20 min. ein N₂-Strom übergeleitet. Die Schmelze wird als Strang aus dem Polykondensationskessel ausgefahren und granuliert. Es werden 43.0 kg farbloses Granulat erhalten.

Das Granulat weist folgende Eigenschaften auf:
- ηᵣₑₗ: = 1.67
- Tₘ: = 219 °C
- [COOH]: = 122 mVal/kg
- [NH₂]: = 31 mVal/kg

Extrusionsfolie (100 µm dick): transluzent, farblos, glatt

### Beispiel 3

Beispiel 2 wird mit derselben 5 Tage schmelzflüssig gelagerten Dodecandisäure wiederholt. Man erhält ein farbloses Granulat mit vergleichbaren Eigenschaften der daraus hergestellten Folie.

### Beispiel B

In einem Kessel (1 m³) mit Ankerrührer werden 500 kg DDS ohne Zusatz von HMD unter Stickstoff aufgeschmolzen und bei 165 °C bevorratet. In einem zweiten Kessel werden 19.728 kg 85 Gew.-%iges HMD (144.30 Mol) unter Rühren innerhalb von 14 Stunden auf 70 °C vorgewärmt. Währenddessen drückt man 33.563 kg schmelzflüssige DDS (Δ 145.88 Mol) aus dem Vorratstank in den Polykondensationskessel und wärmt die Schmelze auf 245 °C vor. HMD wird aus dem zweiten Kessel nachgedrückt. Dabei stellt sich im Polykondensationskessel eine Mischtemperatur von 193 °C ein. Unter Eigendruck wird unter Rühren auf 245 °C (22 bar) aufgeheizt; der Druck wird eine Stunde gehalten. Danach wird unter kontinuierlichem Entspannen auf 265 °C (Normaldruck) aufgeheizt. Anschließend wird während 20 min. ein N₂-Strom übergeleitet. Dann wird das Produkt ausgebracht und granuliert. Es werden 43.2 kg gelbes Granulat erhalten.

Das Granulat weist folgende Eigenschaften auf:
- ηᵣₑₗ: = 1.63
- Tₘ: = 219 °C
- [COOH]: = 124 mVal/kg
- [NH₂]: = 38 mVal/kg

Extrusionsfolie (100 µm dick): transluzent, gelb verfärbt, glatt

### Beispiel C

Beispiel B wird mit derselben zwei Tage schmelzflüssig gelagerten Dodecandisäure wiederholt. Es wird ein stark vergilbtes Granulat mit folgenden Eigenschaften erhalten:
- ηᵣₑₗ: = 1.65
- Tₘ: = 218 °C
- [COOH]: = 116 mVal/kg
- [NH₂]: = 33 mVal/kg

Extrusionsfolie (100 µm dick): transluzent, stark gelb verfärbt, Schlieren

## Patentansprüche

1. Schmelzflüssige, aliphatische Dicarbonsäure mit 8 bis 18 C-Atomen im Kohlenstoffgerüst mit einem Gehalt an primärem Amin von 0.1 bis 3.0 Gew.-% - bezogen auf die Dicarbonsäure.

2. Schmelzflüssige, aliphatische Dicarbonsäure gemäß Anspruch 1 mit 8 bis 13 C-Atomen im Kohlenstoffgerüst.

3. Schmelzflüssige, aliphatische Dicarbonsäure gemäß Anspruch 1, wobei es sich um Korksäure, Sebacinsäure, Dodecandisäure, Azelainsäure, Brassylsäure handelt.

4. Schmelzflüssige, aliphatische Dicarbonsäure gemäß den Ansprüchen 1 bis 3 mit einem Gehalt an primärem Amin von 0.5 bis 2.0 Gew.-%.

5. Schmelzflüssige, aliphatische Dicarbonsäure gemäß den Ansprüchen 1 bis 4, wobei als Amin ein di-primäres Amin enthalten ist.

6. Schmelzflüssige, aliphatische Dicarbonsäure gemäß den Ansprüchen 1 bis 4, wobei als Amin n-Butylamin, n-Hexylamin, n-Dodecylamin, n-Octadecylamin, Cyclohexylamin, Benzylamin enthalten ist.

7. Schmelzflüssige, aliphatische Dicarbonsäure gemäß den Ansprüchen 1 bis 5, wobei als Amin α,ω-Hexamethylendiamin, α,ω-Octamethylendiamin, α,ω-Decamethylendiamin, α,ω-Dodecamethylendiamin, 1.4-Bis(aminomethyl)cyclohexan, 4.4'-Bis(aminocyclohexyl)methan, Isophorondiamin, Triacetondiamin, 2.2.4-Trimethylhexanmethylendiamin, 2.4.4-Trimethylhexamethylendiamin, 2-Methyl-pentamethylendiamin, 1.4-Bis(aminomethyl)benzol, 1.3-Bis(aminomethyl)benzol enthalten ist.

8. Verwendung der schmelzflüssigen, aliphatischen Dicarbonsäure gemäß den Ansprüchen 1 bis 7 als Ausgangsstoff zur Herstellung von (Co)Polyamiden, Polyetheramiden, Polyetheresteramiden.

## Claims

1. A liquid-melt aliphatic dicarboxylic acid having 8 to 18 C atoms in the carbon skeleton with a content of primary amine of 0.1 to 3.0% by weight - based on the dicarboxylic acid.

2. A liquid-melt aliphatic dicarboxylic acid according to claim 1 having 8 to 13 C atoms in the carbon skeleton.

3. A liquid-melt aliphatic dicarboxylic acid according to claim 1, which is suberic acid, sebacic acid, dodecanedioic acid, azelaic acid or brassylic acid.

4. A liquid-melt aliphatic dicarboxylic acid according to any of claims 1 to 3 with a content of primary amine of 0.5 to 2.0% by weight.

5. A liquid-melt aliphatic dicarboxylic acid according to any of claims 1 to 4, which contains as amine a di-primary amine.

6. A liquid-melt aliphatic dicarboxylic acid according to any of claims 1 to 4, which contains as amine n-butylamine, n-hexylamine, n-dodecylamine, n-octadecylamine, cyclohexylamine or benzylamine.

7. A liquid-melt aliphatic dicarboxylic acid according to any of claims 1 to 5, which contains as amine α,ω-hexamethylenediamine, α,ω-octamethylenediamine, α,ω-decamethylenediamine, α,ω-dodecamethylenediamine, 1,4-bis(aminomethyl)cyclohexane, 4,4'-bis(aminocyclohexyl)methane, isophoronediamine, triacetonediamine, 2,2,4-trimethylhexamethylenediamine, 2,4,4-trimethylhexamethylenediamine, 2-methyl-pentamethylenediamine, 1,4-bis(aminomethyl)benzene or 1,3-bis(aminomethyl)benzene.

8. The use of the liquid-melt aliphatic dicarboxylic acid according to any of claims 1 to 7 as starting substance for the preparation of (co)polyamides, polyether-amides and polyether-ester-amides.

## Revendications

1. Acide dicarboxylique aliphatique à l'état fondu, ayant de 8 à 18 atomes de carbone dans son squelette carboné, et contenant de 0,1 à 3,0 % en poids d'une amine primaire par rapport à l'acide dicarboxylique.

2. Acide dicarboxylique aliphatique à l'état fondu selon la revendication 1, ayant de 1 à 13 atomes de carbone dans son squelette carboné.

3. Acide dicarboxylique aliphatique à l'état fondu selon la revendication 1, pour ce qui est duquel il s'agit de l'acide subérique, de l'acide sébacique, de l'acide dodécanedioïque, de l'acide azélaïque, de l'acide brassylique.

4. Acide dicarboxylique aliphatique à l'état fondu selon les revendications 1 à 3, contenant de 0,5 à 2,0 % en poids d'une amine primaire.

5. Acide dicarboxylique aliphatique à l'état fondu selon les revendications 1 à 4, dans lequel l'amine qu'il contient est une diamine primaire.

6. Acide dicarboxylique aliphatique à l'état fondu selon les revendications 1 à 4, qui contient comme amine la n-butylamine, la n-hexylamine, la n-dodécylamine, la n-octadécylamine, la cyclohexylamine, la benzylamine.

7. Acide dicarboxylique aliphatique à l'état fondu selon les revendications 1 à 5, qui contient comme amine l'α,ω-hexaméthylènediamine, l'α,ω-octaméthylènediamine, l'α,ω-décaméthylènediamine, l'α,ω-dodécaméthylènediamine, le 1,4-bis(aminométhyl)cyclohexane, le 4,4'-bis(aminocyclohexyl)méthane, l'isosphoronediamine, la triacétonediamine, la 2,2,4-triméthylhexaneméthylènediamine, la 2,4,4-triméthylhexaméthylènediamine, la 2-méthyl-pentaméthylènediamine, le 1,4-bis(aminométhyl)benzène, le 1,3-bis(aminométhyl)benzène.

8. Utilisation de l'acide dicarboxylique aliphatique à l'état fondu selon les revendications 1 à 7 comme matière de départ pour la préparation de (co)polyamides, de polyétheramides, de polyétheresteramides.
